Europäisches Patentamt

European Patent Office

Office européen des brevets

(11) Publication number: **0 148 161**
A2

# EUROPEAN PATENT APPLICATION

(21) Application number: **85300003.2**

(22) Date of filing: **02.01.85**

(51) Int. Cl.⁴: **C 12 M 3/00**

(30) Priority: **03.01.84 US 567886**

(43) Date of publication of application:
**10.07.85 Bulletin 85/28**

(84) Designated Contracting States:
**BE DE FR GB IT LU NL**

(71) Applicant: **E.I. DU PONT DE NEMOURS AND COMPANY
1007 Market Street
Wilmington Delaware 19898(US)**

(72) Inventor: **Root, Richard Terry
2855 E. Oakland Drive
Wilmington, Delaware 19808(US)**

(74) Representative: **Hildyard, Edward Martin et al,
Frank B. Dehn & Co. Imperial House 15-19 Kingsway
London WC2B 6UZ(GB)**

(54) **Cell culture chamber.**

(57) A closed cell culture chamber of which at least one wall (14) comprises liquid impermeable, transparent ionomer film, said chamber having an access port (12) for admission of fluid thereto.

The apparatus may include an edge support (32, 34) to support and tension the film.

Fig. 4.

EP 0 148 161 A2

Croydon Printing Company Ltd

## "CELL CULTURE CHAMBER"

This invention relates to a cell culture chamber.

Recently it has been demonstrated that cell culture can be performed in gas-permeable, liquid-impermeable thin-film containers with no enclosed air space if adequate gas transport through the container walls is achieved. Flexible, film-walled containers made of Teflon® FEP and other films have been used for culturing purposes. Flexing of such containers has been shown to facilitate the release of attachment-dependent cells for harvesting. In addition, such containers can be sealed to maintain sterile conditions during cell growth.

Various approaches to growing cells in film-based containers are described in the patent and technical literature. Thus, U.S. Patents 3,887,436, 3,948,732 and 4,033,825, issued to Instrumentation Laboratory, describe the use of an elongated, flattened, tubular chamber supported on a reel; the chamber being formed of FEP Teflon® film and taking a shape, when filled with medium, as defined by the reel. This tubular chamber is used for continuous as opposed to batch-type cultures. Two other U.S. Patents 3,873,423 and 3,941,662 issued to Munder, et al., describe a Teflon® thin film chamber sealed at the edge and supported on a wide mesh screen. While permitting batch-type cultures, the Munder, et al., chamber is not easily handled. A paper by Munder, et al., appearing in FEBS Letters 15 (#3) 191 (1971) describes the use of a film bag for batch culture of cells and teaches turning over the two-sided film container to permit growth of cells on both sides of the membrane chamber, thereby enhancing cell production.

Additional publications describing batch type culture growth in plastic bags are:

1) J. W. M. van der Meer, et al., Cellular Immonol. 42, 208 (1979);

2) J. W. M. van der Meer, et al., Immunology, 47, 617 (1982);

3) M. D. Jensen in TCA Manual, Vol. 2, No. 1, p. 265 (1976);

4) M. D. Jensen et al., Exp. Cell. Res., 84, 271 (1974); and

5) P. G. Munder, et al., Eur. J. Immunol., 3, 454 (1973).

Finally, U.S. Patent 4,142,940 issued to Munder, et al., describes a disposable cup (tissue culture vessel; petri dish) in which a pair of plastic rings lock a gas permeable membrane which serves as the dish bottom (on which cells grow) free of wrinkles, folds, or undulations.

Regardless of the support used, films used for such cell growth containers should be heat sealable, have relatively high puncture, tear and tensile strengths, be adequately permeable to oxygen and carbon dioxide for the cell line, be relatively impermeable to liquid, non-toxic to the cells, and be highly transparent. The film should be relatively inexpensive and available in relatively thin thickness to maintain sufficient transparency for viewing with the eye or a microscope. Finally the film should facilitate the attachment of cells in growth media. The films typically used for cell growth containers in the past do not meet all of those criteria. These films have included Teflon® FEP film, polyethylene film and others.

In one aspect, our invention provides a closed cell chamber of which at least one wall comprises a sheet of a gas permeable, liquid impermeable, transparent ionomer film, said chamber having an access port for admission of fluid thereto. Such films can be designed to have the requisite high tensile and puncture strengths and are amenable to surface modification if desired.

In one embodiment, the chamber comprises two sheets of said inomer film, the sheets being edge sealed together.

An ionomer film with an oxygen permeability of between about 50 and 800 cc-atmospheric oxygen/100 in$^{-2}$-24 hours (3 x 10$^{-11}$m/s Pa to 50 x 10$^{-10}$m/s Pa) ASTMD-1434, water permeability $\leq$ 150 g/100 M$^2$-hr-mil (100°F 90% RH), tensile strength $\geq$ 1500 psi (10.3 MPa) ASTMD-882, a puncture strength of at least about 100 (ASTM-1709) and a haze of about 5 or less (ASTMD-1003) is suitable for this purpose. Such type of film is clear, readily available, reliably heat sealable, chemically inert, has good toughness (adequate tensile and puncture strengths), and has functional groups on its surface which facilitates its surface modification if desired.

A satisfactory ionomer film is a film 0.1-10 mils (24-250 µm) made from Du Pont's Surlyn® 1601 polyethylene ionomer. It will be clear to those skilled in the art that equivalent ionomer films may be made from ethylene acid copolymers containing an optional third comonomer. The acids can be acrylic,

methacrylic, maleic acid, maleic acid half esters, fumaric and half esters, and itaconic acid. The optional third comonomer can be a softening comonomer such as propylene, acrylate esters, methacrylate esters, vinyl esters, vinyl ethers, and t-butyl ethylene.

Other materials that can be used could be carbon monoxide, vinyl chloride or bromide, styrene, acrylonitrile, and methacrylonitrile. The acid copolymers can be neutralized with any elements from group Ia or IIa or other cations which are not toxic to the cell to be grown. In addition, amines such as, ammonia, triethylamine, ethylene diamine and hexamethylene diamine could be used to form ionomers. The acid levels should be from about 1-30% by weight (0.3-12.5 mole %) based on methacrylic acid equivalents. The softening comonomer or third comonomer can be present up to 0-25% weight % (or 7.5 mole %) based on butyl acrylate. The remainder of the polymer should be ethylene. These acid copolymers should be neutralized with the described ions to a level of 0-90% neutralization. The ionomers may be prepared directly or by graft polymerization.

Preferably two sheets of an ionomer film are edge heat sealed together to provide a closed chamber. An access port is formed in the chamber to provide fluid access thereto. This chamber may be provided with an edge support, described in the copending Root and Long application, having first and second frame members adapted to interengage each other and the edges of the sheets to support and tension the sheets therebetween.

One edge support that can be used for the chamber is rectangular in shape with the first frame member defining a groove in its upper surface and the second frame member defining a tongue in its lower surface adapted to engage the groove and grip and tension the sheets.  This is possible because of the high tensile and tear strength of the ionomer film. The frame member may define a slot in its upper surface and the sheets define a corresponding entry port at a location adjacent to the slot.  The edge support is formed of a relatively rigid plastic and the frame members are integral and connected by a living hinge.

### A Brief Description of the Drawings

Other features and many of the advantages of the invention will readily become apparent from the following detailed description when considered in connection with the appended drawings in which:

Figure 1 is a plan view of a cell culture container constructed of an ionomer film in accordance with this invention;

Figure 2 is a side elevation view of the container in Figure 1;

Figure 3 is an end elevation view of the container in Figure 1;

Figure 4 is a pictorial view of the container;

Figure 5 is a fragmentary cross-sectional view of the container taken along the section lines 5-5 of Fig. 1;

Figure 6 is a partially exploded view of a cell culture container constructed using an ionomer film in accordance with another embodiment of this invention;

Figure 7 is a pictorial view of the frames of Fig. 5 in an assembled form; and

Figure 8 is a fragmentary cross-sectional view of the frames of Fig. 6 about to be assembled.

## Detailed Description of the Preferred Embodiment

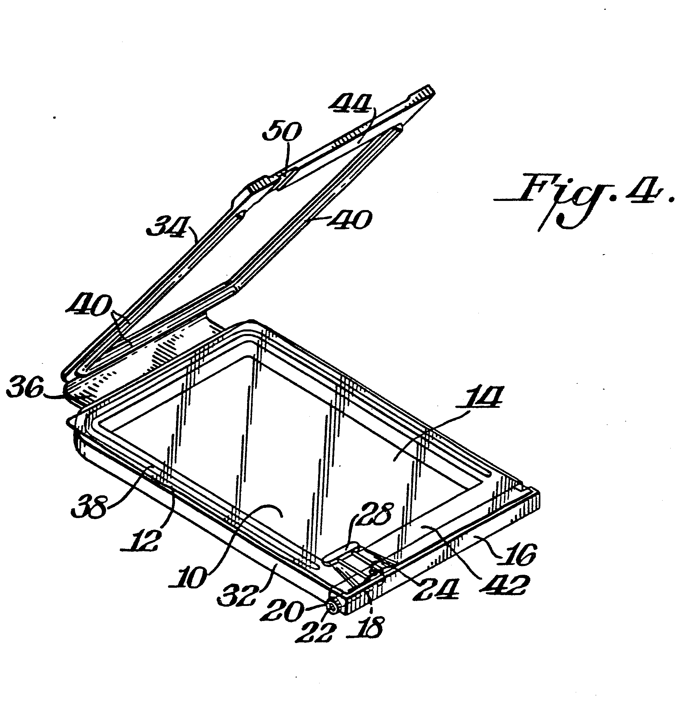

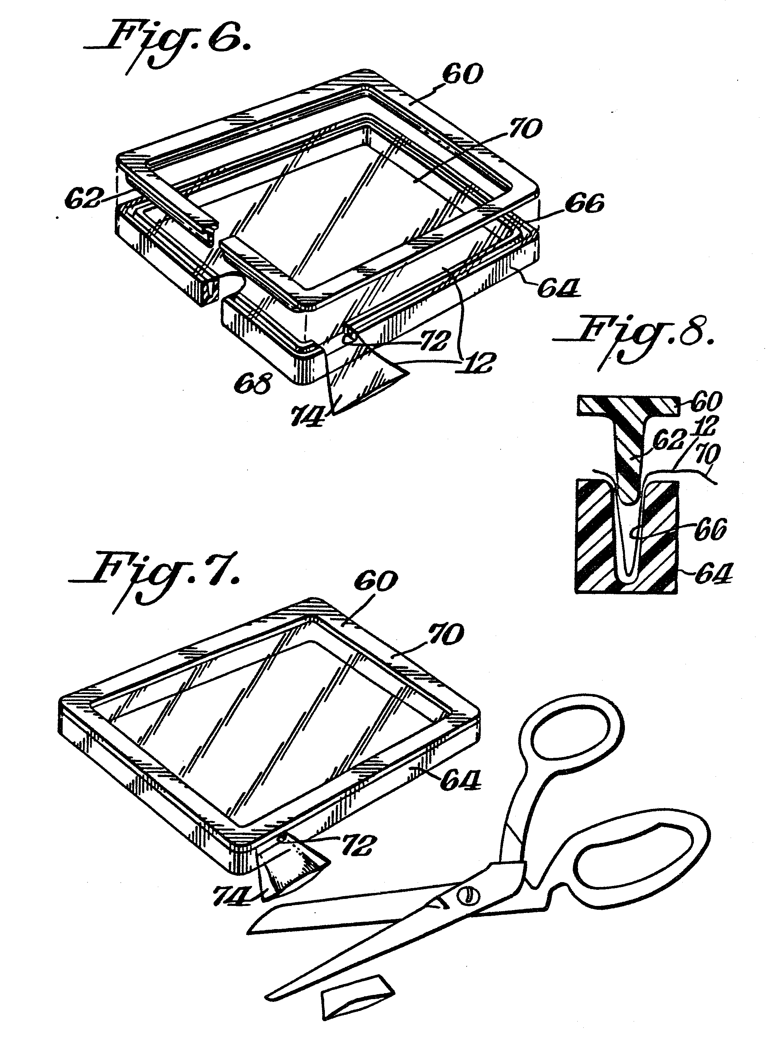

A closed cell culture container constructed with a frame to hold an ionomer film chamber 10 of this invention is best seen in Figure 4. In this figure, the chamber 10 is formed of two sheets of a thin ionomer film material that are heat sealed about their edges as at 12 to provide the closed chamber 10. Regardless of the frame support used, the ionomer film used for such cell growth containers should be heat sealable, have a relatively high puncture, tear and tensile strength to safely contain the cultures under normal handling, be adequately permeable to oxygen and carbon dioxide to support the cell line, be relatively impermeable to liquid, non-toxic to the cells, and be highly transparent. The film should be relatively inexpensive and available and maintain sufficient transparency for viewing with the eye or a microscope. Finally the film should be amenable to control of attachment of cells in growth media. The film surface can be chemically modified to enhance or abrogate cell adhesion, as desired. An example of such a modification would be the covalent attachment of a specific biopolymer (fibronectin, laminin, chondronectin or albumin) which has a known function in cell to surface adhesion. The process through which the novalent attachment could be achieved with an ionomer film is the conversion of the acid OH groups to acyl chloride groups, as described in United Kingdom Patent 1,048,914 or in U.S. Patent No. 3,310,518 issued to Daniel Maloney (see particularly Examples I and II).

The films typically used for cell growth containers in the past do not meet all of those criteria. The ionomer film used in this invention may have a thickness in the range from 1 to 10 mils. (24-250 μm) with 2-6 mil. (48-150 μm) thickness being preferred.

An ionomer film with an oxygen permeability of between about 50 and 800 cc-atmospheric oxygen/100 $in^{-2}$-24 hours (3 x $10^{-11}$ m/s Pa to 50 x $10^{-10}$ m/s Pa) ASTMD-1434, water permeability ≤ 150 g/100 $M^2$-hr-mil (100°F 90% RH), tensile strength ≥ 1500 psi (10.3 MPa) ASTMD-882, a puncture strength of at least about 100 ASTM 1709 and a haze of about 5 or less (ASTMD-1003) is suitable for this purpose. Such type of film is clear, readily available, reliably heat sealable, chemically inert, has good toughness (adequate tensile and tear strengths), and has functional groups on its surface which facilitates its surface modification if desired. An example of a satisfactory ionomer film in the specified thickness range is a film made from Du Pont's Surlyn® 1601 polyethylene ionomer.

It will be clear to those skilled in the art that equivalent ionomer films may be made from ethylene acid copolymers containing an optional third comonomer. The acids can be acrylic, methacrylic, maleic acid, maleic acid half esters, fumaric and half esters, and itaconic acid. The optional third comonomer can be a softening comonomer such as propylene, acrylate esters, methacrylate esters, vinyl esters, vinyl ethers, and t-butyl ethylene. Other material that can be used could be carbon monoxide, vinyl chloride or bromide, styrene, acrylonitrile, and methacrylonitrile. The acid copolymers can be neutralized with any elements from group Ia or IIa or other cations which are not toxic to the cell to be grown. In addition, amines such as, ammonia, triethylamine, ethylene diamine and hexamethylene diamine could be used to form ionomers. The acid levels should be from 1-30% by weight (0.3-12.5 mole %) based on methacrylic acid equivalents. The softening comonomer or third comonomer can be present up to (0-25 weight % - 7.5

8

mole %) based on butyl acrylate. The remainder of the polymer should be ethylene. These acid copolymers should be neutralized with the described ions to a level of 0-90% neutralization. The ionomers may be prepared directly or by graft polymerization.

Preferably, one common edge of both films 14 is heat sealed to one surface of a header 16. To aid in heat sealing, the header 16, preferably may be formed of an ionomer resin such as Surlyn® 1601, and is molded in the rectangular, rod-like configuration shown with an end bore 18 to accommodate a valve insert 20 or septum (not shown). Other header materials that may be used include high density polyethylene, polypropylene, polyvinyl chloride or polystyrene. These require the use of an adhesive as they are not heat sealable to the film. The valve insert 20 has a central conduit 22 and a radial bore 23 (Fig. 5) communicating with the conduit 22 and with an orifice 25 formed in the header 16. A hole 27 is formed in one of the films 14 that is immediately contiguous to the header 16 to provide access to the interior of the chamber. This hole communicates with the orifice 24 and permits fluids to be introduced into or withdrawn from the chamber. By turning the valve insert 20, the fluid path is opened or closed.

The header may also have a septum adapted to accomodate a syringe or like device. A support paddle 28 may be secured to the header and introduced into the bag to maintain the two films 14 separated and facilitate the passage of fluid to and from the interior of the chamber 10 formed by the films 14. Alternatively, a bead in the chamber may be used for this purpose. A funnel or pipette or syringe may be used to introduce fluids through the conduit 22. In this event, the conduit 22 is adapted as by tapering to receive a pipette or other funnel-like devices for the transport of fluids to and from the chamber.

The contacting surfaces of the film forming the chamber 10 are deblocked, to reduce their tendency to stick together. Deblocking is accomplished by dusting the contacting film surfaces with a dry powder to create passages for fluid to pass between the films. The powder used should not (a) be toxic to the cells, (b) cause significant alteration of the growth characteristics of the cells, (c) adversely affect the character of the film surface or cause an interaction of the cells therewith, (d) leave a residue that interferes with the clarity of the films, or (e) change the pH or ionic strength of the nutrient.

A suitable material for this purpose is powdered sucrose. Other sugars or soluble chemical materials meeting the above criteria may be used. These include glucose, other sugars, metabolic intermediates such as citric acid or amino acids. Thus used, the deblocking agent, after it has performed its deblocking function, simply dissolves in the nutrient when it is added.

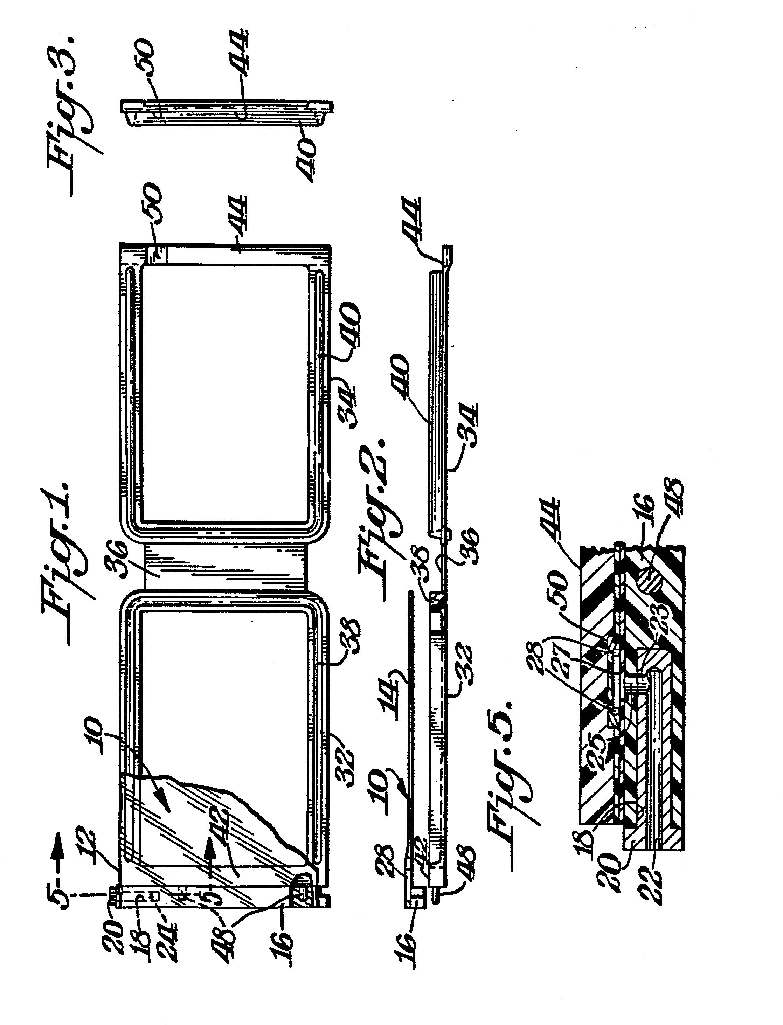

Although the frame is not a part of this invention, there may be seen in Figs. 1 to 4, a container formed of a pair of edge support frames 32, 34 which together with the header 16 support the chamber 10 of this invention in a tensile manner with the films stretched between the frame and header to provide a flat, smooth, even surface for cell culture. The edge support for the chamber includes a first or lower frame 32 and a second or upper frame 34 connected together by an integrally formed living hinge 36. The frames and hinge are formed by a single molding operation and preferably are formed of a high-density polyethylene, polypropylene, polyvinylchloride or other plastic that will provide a relatively rigid support structure that is capable of flexing at the living hinge. As may be seen, the upper and lower frames 34, 32 are generally rectangular with the lower frame 32 having a groove 38 formed in its upper surface along three sides and the upper frame 34 having a tongue member 40 formed in its lower surface. The remaining sides 42 and 44 of the respective frames do not have a groove, but the lower frame 32 is provided with a pair of horizontally extending pins 48 which are adapted to engage corresponding receptacles in and removably mount the header 16 (Fig. 4). The header 16 is shown in phantom in Figs. 1 and 2. A slot or channel 50 is formed in the side 44 to permit fluid access to the film chamber when it is positioned within the frame. The paddles 28 extend through this slot 50 to maintain the fluid passage open particularly during suctioning to withdraw fluid from the chamber.

To use, or to construct a cell culture container, with removable chambers, one need merely select a header 16 with the film chamber attached, position the header over the pins 48 of the lower frame member and the film over the remainder of the lower frame 32 so that it covers the groove 38. The upper frame 34 now is folded at the living hinge 36 so that the tongue member 48 is introduced into the groove thus engaging the film and pressing the film into the groove, thereby tensioning the film about the frame member. Thus positioned, the chamber provides a relatively flat, even surface with reduced sag and which surface can be readily viewed by a microscope to ascertain cell culture growth. Because of the tensioned flatness of the high strength ionomer films 14, fluids in the chamber are more uniformly and evenly supported than with other materials. The overall thickness of the cell culture container is small, maintaining good fluid and air contact with the cell culture. Top and bottom visual or microscopic observation is facilitated due to the optical clarity of the ionomer films. Such films are highly permeable to gases needed for cell culture yet is impermeable to liquids, thus holding the nutrient media.

Growth media and cell cultures may be introduced through the conduit 22 and thereafter the valve insert turned so that the orifice 24 is closed. Following the use of a particular chamber, the upper frame 34 may be pulled away from the lower frame thereby releasing the film. The header 16 may now be removed from the pins 48 carrying with it the thin film chamber 10. The chamber may now be flexed as desired to assist in releasing the cells.

In another embodiment of the invention, as seen in Fig. 6, a chamber is constructed of an ionomer film of the type described above without the use of a header. This chamber is depicted as being mounted in a support frame although this is not necessary to the invention. The chamber may be used alone. In Fig. 6, a rectangular upper frame 60 has a raised tongue 62 along the lower surface of each side of the frame. Likewise a rectangular lower frame 64 has a groove 66 formed in each side of its upper surface adapted to receive the tongue 62 of the upper frame. The frames may be constructed of the same materials as the frames illustrated in Figs. 1-4. Next a pair of ionomer films are deblocked and edge sealed as previously described to provide a closed chamber that will fit over the frames. A slot 65 is formed in one edge of the lower frame 64. The films 70 are edge sealed to define an exit or an access flap 74 which may be tapered and shaped to accommodate a pipette or similar device to provide access to the interior of the chamber formed by the films 70. This flap access 74 may be heat sealed to maintain sterility of the chamber. The flap is then cut to permit access and again heat sealed or clamped to maintain sterility.

There is depicted in Figure 8 the manner in which the mounting of the chamber in the frame is accomplished, i.e., the edge sealed films 70 are placed over the lower frame 64 and the upper frame 60 engaged with the lower frame 64, thereby gripping and tensioning the ionomer films 70. The flap 74, of course is positioned in the slot 72 to permit fluid access to the chamber. If needed, the tongue 62 at its mating location with the slot 72 may be removed or reduced in height to maintain adequate access for fluids through the flap. When this is accomplished, the chamber is formed as depicted in Fig. 7 with the ionomer films in a stressed, taut condition providing a relatively smooth surface that can be used for cell culturing purposes as previously described. Observation of the cells is facilitated by the convenient container thus formed. The flap may be replaced by a tube which can be connected by other tubes using a sterile docking device of the type described in Spencer's U.S. Patent 4,369,779 if desired.

### Example A

Successful attachment and growth of 3 representative fibroblastic cell lines (VERO, MRC-5 and WI-38) and 1 epitheliod line, MDCK, has been documented by photomicrography on 2-mil Surlyn® 1601 grade film mounted as the support surface in a conventional culture dish arrangement. In addition, a comparison VERO cell line was grown on a standard 25 square continuity polystyrene culture flask. Minimum Essential Medium (Eagle) supplemented with 5% fetal bovine serum (FBS) and nonessential amino acids (MEM$^+$) was used to support VERO cell growth, while Iscove's Modified Dulbecco's Medium containing 10% FBS was used for the other 3 cell lines. Standard cell culture techniques were employed throughout.

The maximum cell yield for the VERO line was determined to be ~7 x 10$^5$ cells/cm$^2$, a little more than twice the maximum density measured on polystyrene under otherwise identical culture conditions.

Example B

The VERO cell line was cultured inside sealed bags constructed from 2-mil thick Surlyn® 1601 grade film, having a (single-face) growth area of 76 cm$^2$. The volume of MEM$^+$ employed per bag was 0.15 mL/cm$^2$, as it was in Example A and the initial inoculum contained ~ 7 x 10$^3$ cells/cm$^2$. The confluent cell density obtained, employing otherwise standard conditions, was found to be ~4.2 x 10$^5$ cells/cm$^2$.

Example C

VERO fibroblastic cells were cultured inside sealed bags constructed from Surlyn® 1601 grade film of varying thicknesses between 0.8 and 4.5 mils. The available (single-face) growth surface area was again 76 cm$^2$ with other conditions as specified in Example B, except that no refeeding of the bags was performed. The growth curves were carried out over 8 days, with an essentially identical maximum cell yield of ~1.6 x 10$^5$ cells/cm$^2$ achieved for all film thicknesses at day 5 of the study. The calculated mean generation time (MGT) increased smoothly with increasing film thickness from 7.8 hours to 11.8 hours (on polystyrene, the

MGT for VERO is 11.4 hours). The plating efficiency also increased smoothly with increasing film thickness from 2.1% to 14.3%.

The ionomer films may be used to form any shape of chamber desired and the chamber may have any type of port for the introduction of nutrients and cells. The important features are the advantages afforded by the chamber for cell culture purposes because of the use of the ionomer film with unique properties.

- 15 -                    0148161

Claims:

1. A closed cell culture chamber of which at least one wall (14) comprises a sheet of a gas permeable, liquid impermeable, transparent ionomer film, said chamber having an access port (22) for admission of fluid thereto.

2. A chamber according to claim 1 which comprises two sheets of said ionomer film, the sheets being edge sealed (12) together.

3. A chamber according to claim 1 or 2 including an edge support (32, 34) to support and tension said film.

4. A chamber according to claim 3 wherein said edge support comprises first and second frame members (32, 34) adapted to interengage each other and the edges (12) of said sheet.

5. A chamber according to any of the preceding claims in the form of a rectangular chamber having four sides, three sides being defined by an edge seal (12) between two sheets of said ionomer film, the fourth side being defined by a resin header (16) sealed to the fourth side of the sheets, the header providing access to the interior of the chamber.

6. A chamber according to any of the preceding claims wherein the ionomer film has a thickness in the range of 24-250 $\mu$m.

7. A chamber according to any of the preceding claims wherein the ionomer film has an oxygen permeability of between $3 \times 10^{-11}$ and $50 \times 10^{-10}$ m/s Pa.

8. A chamber according to any of the preceding claims wherein the ionomer film has a tensile strength greater than 10.3 MPa and a puncture strength of at least about 100 as defined in ASTM 1709.

9. A chamber according to any of the preceding claims wherein the ionomer film has a haze of about 5 or less as defined in ASTM D1003.

10. A chamber according to any of claims 1 to 5 wherein the ionomer film has a thickness in the range 48-150 µm; has an oxygen permeability of between $3 \times 10^{-11}$ and $50 \times 10^{-10}$ m/s Pa; has a tensile strength greater than 10.3 MPa, a puncture strength of at least about 100 as defined in ASTM 1709; and a haze of about 5 or less as defined in ASTM D1003.

11. A chamber according to any of the preceding claims wherein said ionomer film comprises a copolymer of ethylene with 0.3-12.5 mole % (based on methacrylic acid equivalents) of acrylic acid, methacrylic acid, maleic acid or a half ester thereof, fumaric acid or a half ester thereof or itaconic acid, if desired with a further comonomer.

12. A chamber according to claim 11 wherein said further comonomer comprises up to 7.5 mole% (based on butyl acylate) of propylene, acrylate esters, methacrylate esters, vinyl esters, vinyl ethers or t-butyl ethylene.

13. A chamber according to any of the preceding claims wherein the surface of said ionomer film is chemically modified to enhance or abrogate cell adhesion.

14. A method of culturing a cell line which comprises introducing a viable cell line and a suitable culture medium into the chamber defined in any of claims 1 to 13 through said access port, closing said access port, and allowing growth of said cell line to take place utilising gases permeating through said ionomer film.

Fig.1.

Fig.3.

Fig.2.

Fig.5.

1/3

0148161

0148161

Fig. 4.

*Fig.6.*

60

70

62

66

64

72

12

68

74

*Fig.8.*

60

62 12

70

66

64

*Fig.7.*

60

70

64

72

74